# EUROPEAN PATENT APPLICATION

(11) **EP 1 406 089 A1**
(43) Date of publication of application: **07.04.2004**
(21) Application number: 02022124.8
(22) Date of filing: 02.10.2002
(51) Int. Cl.: G01N 33/532, G01N 33/68, G01N 33/58

(54) **A method for selectively staining water soluble protein using reactive dye**

(71) Applicant: Taiwan Unison Biotechnology Co. Ltd., Hsichu (TW)
(72) Inventor: Hsiung, Kuang-Pin, Hsinchu (TW); Hseu, Tzong-Hsiung, Hsinchu (TW); Yao, Wen-Fa, Hsinchu (TW); Liu, Yung-Chuan, Hsinchu (TW)
(74) Representative: Becker Kurig Straus

(57) **Abstract**

A method of selectively staining the water-soluble protein in which a commonly used reactive dye binds to the protein by reacting with a carboxyl group, amino group, and thiol group from the amino acids that make up the protein. When the specimen to be stained is an antibody, the reaction is adjusted to occur in an acidic environment so as to promote reacting the reactive dye with the carboxyl groups of the amino acids of the antibody and the carboxyl group of oligosaccharide conjugated to the constant region of the antibody. The staining reaction occurs in an alkaline solution to produce a more intense staining for the protein, without using the antibody as a marker ligand. After the protein is stained with the reactive dye, an unreacted portion of portion dye molecules is separated from the stained protein. The stained protein is purified and the stained protein is further freeze-dried.

## Description

### FIELD OF INVENTION

The present invention relates to the methods for staining protein. More particularly, the present invention relates to methods for both selectively staining a glycosylated protein either by directly using reactive dye or via staining the carrier molecules prior to the carrier is conjugated to target protein selectively and non-selectively staining the water soluble protein for different analytical application.

### DESCRIPTION OF RELATED ART

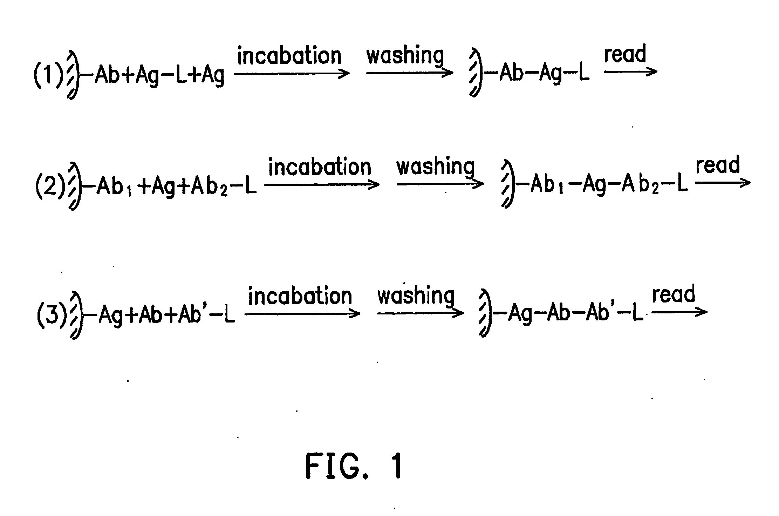

The immunological analysis or simply known as immunoassay has been used widely for practical application in different fields, such as clinical diagnosis, environmental monitoring, agricultural product analyzing, food processing control and scientific research. Regardless of the types of the immunoassay, a small organic compound in the test specimen or a biological polymer such as a protein molecule, a nucleic acid molecule or a polysaccharide molecule in the specimen to be assayed usually needs to label for qualitation and quantitation in the analysis. For example, when the test specimen is an antigen(Ag) or antibody(Ab), logic analysis designs such as those illustrated in Fig 1 can be developed depending on cost and availability of antigen and antibody and so on. Ab1 and Ab2 are antibodies against the test specimen showing two different characteristics, while L is a marker ligand. Ab' is the secondary antibody against first animal species antibody. When the ligand is a radioactive element, the immunoassay involved with such ligand is known as a radioimmunoassay (RIA). With an enzyme serving as the ligand, such immunoassay is known as an enzyme immunoassay (EIA). Furthermore, when the immunoassay utilizes a fluorophor or luminescent compound as the ligand, such immunoassay is termed a fluorescent immunoassay (FIA) or luminescent immunoassay (LIA). In general, the immunoassay with different ligands provide different quantification methods and thus are given different names. Nevertheless, the immunoassays described above are the major immunoassay approaches commonly used.

Ever since the RIA has been widely practiced in fifties, it was recognized for its benefits, such as sensitivity, and low cost. However, issues regarding to operation safety and waste disposal have then restricted further development of the RIA. So, the EIA developed in early seventies had taken most of market from the RIA. Unfortunately, the EIA suffers from low stability and sensitivity, as well as a long incubation time. Both FIA and LIA known for their higher sensitivity were developed to substitute the role played by the RIA. But, once both of the immunoassay methods are modified to incorporate into a hand-free, automated immunoassay analyzer, the large size and high cost incurred would naturally limit such immunoassay techniques from benefiting the general society, in application such as point-of-care testing(POCT), point-of need testing(PONT) and so on.

In the eighties, people have attempted to use diffusible dye for making micro-to-nano range particles that adsorb antibody, thereby developing another immunoassay technique, known as dye immunoassay(DIA). But, this immunoassay has not been widely used due to process factors such as difficulty in controlling particle size distribution, shape and different binding ability of the diffusible dyes to the same protein. And DIA thus prepared is generally considered as less sensitive than other immunoassays. Moreover, poor stability of the dye particles after Ab absorption further poses problem that needs to be resolved before its wider applicability can be appreciated.

The reactive dye, also known as active dye, was developed in 1956 and designed to bind a dye molecule through a covalent bond to the hydorxy group( -OR) of the cotton fiber. The commonly used reactive dye is basically classified into two groups, such as a reactive dye of trazine series that concerns with a nucleophilic substitution, and a reactive dye of vinyl sulfone series that concerns with nucleophilic addition. Although the design of the dye was originally intended for staining cotton fiber, the dye has also been used for staining wool and silk by reacting with the aniino(-NH2) and thiol(-SH) groups due to the two nucleophilic reactions described above. Typically, when materials such as wool, silk, and cotton are stained using the reactive dye, the reaction is usually kept at a pH of above 10.5, at temperature of 60~100°C as well as a large amount of salt. The purpose of adding the salt includes (1) to promote expansion of the all textile materials so that color is distributed more uniformly, (2) to speed up the staining reaction, (3) to obtain a high staining yield with a low production of waste so as to satisfy the requirement of environmental care, (4) to dissociate proton ion from the hydroxy group and thiol group in an alkaline environment, while the charge of amino group is neutralized, so as to significantly increase electronegativity of lone paired electron from the amino functional groups of the amino acids. As a result, the electrophilic reaction of the dye molecule is improved. The electrophilic activity of reactive dyes toward protein's amino acid functional groups rated as -NH2>-SH>-OH, in the regular fabric staining condition.

As illustrated in Figure 1, reactive dye can be utilized as ligand to stain either antibody or antigen for an designated immunoassay process. However, the antigen binding epitopes of antibody molecule are the amino end of the protein. Therefore, the antigen-antibody binding affinity can be abolished or partially abolished if the antigen binding epitopes of antibody molecule are reacted or/and sterically blocked from closed by amino acid electrophilic functional groups with dye molecules. Once, these kinds of reaction happen then the sensitivity of immunoassay using dyed antibody produced by this reactive-dyeing approach will be severely hindered.

Also, the harsh reaction conditions for fabric staining when dealing with reactive dyes should naturally drive the fragile biological molecules denature.

Therefore, selective protein dyeing particularly antibody dyeing methods have to be developed to further increase the sensitivity so is the practicability of the approach. Also, a moderate reaction conditions for staining protein and used as marker molecule as seen in Figure 1, had to be optimized.

### SUMMARY OF THE INVENTION

The present invention provides i). a method for selectively staining the saccharificate moiety and the hydroxy group of amino acids of antibody molecule or the hydroxy group of amino acids of a glycosylated protein using reactive dye directly, and ii). a method of which the natural or synthetic water-soluble polymeric "carrier" is first stained then conjugated selectively to the saccharificated moiety and the hydroxy group of amino acids of antibody or the hydroxy group of amino acids of a glycosylated protein. Both methods have benefit in terms of cost and sensitivity when applied to an immunoassay protocol. To achieve these and other advantages, and in accordance with the purpose of the invention, as embodied and broadly described herein, the invention provides the methods of antibody active staining using the reactive dye that produces a electrophilic reaction with the protein nucleophilic function groups. In a "direct" staining reaction, the reactive dye can bind to antibody by reacting preferably with the hydroxy group than thiol and amino side chain functional groups among the amino acids that make up the antibody, under the appropriate reaction conditions. After the protein is stained with the reactive dye, the dye molecule that does not undergo any reaction would be separated from the stained protein. The stained protein can then be purified to obtain a higher purity, while the stained protein can be further freeze-dried to improve its storage. In the "carrier" approach, the biologically-inert macromolecules were selected and heavily stained and then reacted with peroidate activated. oligosaccharide moiety of the glycosylated molecule. Both of the approaches selectively alleviate the reaction of dye molecules with amino groups at and nearby antigen-binding epitopes located at the amino end of antibody molecules and increase the specificity and sensitivity of the immunoassay when the stained antibody is thus used in an assay protocol.

The present invention therefore, also provides the methods of staining any oligosaccharide containing protein and regular protein when it is considered as analyte in the specimen in a competitive protocol using these reactive dyes that are commonly used in the textile industry.

When the specimen to be directly stained is an antibody, the reaction is adjusted to occur in an acid environment, in the presence of certain salt concentration, so as to promote reacting the reactive dye with the hydroxy group from the amino acids that make up the antibody and the hydroxy group from the oligosaccharide conjugated to the constant region of the antibody. However, if the specimen to be stained is a non-antibody protein or a non-glycosylated protein, the staining reaction can occur in an optimized alkaline solution to produce a more intense staining of the protein for better immunoassay sensitivity.

It is to be understood that both the forgoing general description and the following detailed description are exemplary, and are intended to provide further explanation of the invention as claimed.

### BRIEF DESCRIPTION OF THE DRAWINGS

The accompanying drawing are included to provide a further understanding of the invention, and are incorporated in and constitute a part of this specification. The drawing illustrate embodiments of the invention and, together with the description, serve to explain the principles of the invention. In the drawings,

Fig. 1 is an illustrative diagram showing the flow chart of different immunoassay protocols

Fig. 2 is a double immunodiffusion diagram showing antibody-antigen precipitation lines produced from the serial diluted myoglobin stained with a Amido black against unstained rabbit anti myoglobin antibody on the agarose substrate; (a), myoglobin against rabbit anti myoglobin stained with Amido black, 2X ,4X ,8X ,16X, 32X, & 64X, (b), myoglobin against rabbit anti myoglobin stained with Amido black, 128X, 256X, 512X, 1024X, 2048X, 4096X.

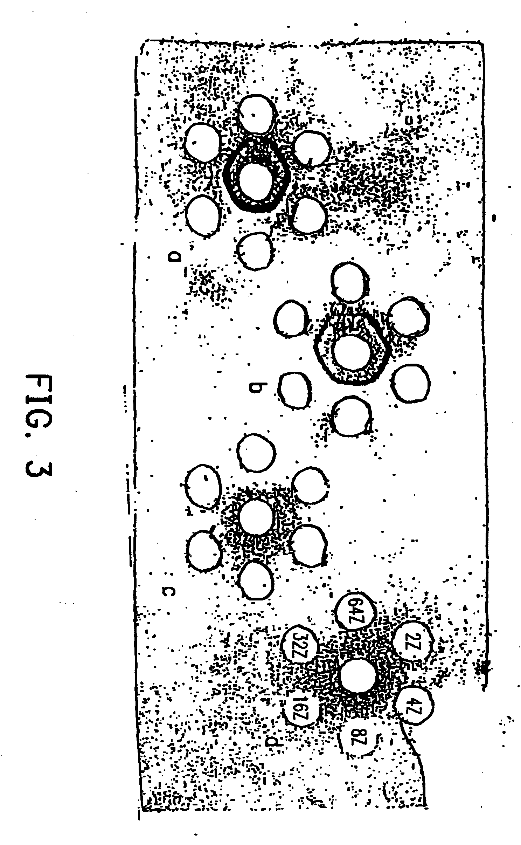

Fig. 3 is a double immunodiffusion diagram showing antibody-antigen precipitation lines produced from the serial diluted of rabbit anti myoglobin polyclonal antibody stained with Procion red MX-8R against myoglobin on an agarose substrate; (a), dye-loaded "carrier" staining antibody, Sephadex G-150 purified, (b), "direct" acid-staining antibody, Sephadex G-25 purified, (c), unstained replaced "direct" acid-stained rabbit anti myoglobin antibody and Procion red MX-8R replaced myoglobin, (d), unstained rabbit anti myglobin antibody against myoglobin plus Procion red MX-8R, in a dilution series of 2, 4, 8, 16, 32 and 64.

Fig. 4 is a spectrogram of bovine albumin stained with the reactive dye Procion red MX-8R and purified with Sephadex G-25 column; and

Fig. 5 is a calibration curve for myoglobin when using "direct" acid stained myoglobin antibody in sandwish-type immunochromatograph.

### DESCRIPTION OF THE PREFERRED EMBODIMENTS

Reference will now be made in detail to the present preferred embodiments of the invention, examples of which are illustrated in the accompanying drawings. Whenever possible, the same reference numbers are used in the drawings and the description to refer to the same or like parts.

It was known that an protein has played an important role in a typical immunoassay. Particularly, immunoglobulin has on its constant region (Fc region) and variable region (Fv region), and the oligosaccharide molecules that occupy a 2~3% of total molecular weight of immunoglobulin G, mainly spread around on constant regions of the molecule. Whereas an amino group from both the heavy chain and light chain of the antibody constitute the major part of Fv region, the antigen-binding epitope. As a staining reaction is allowed to occur randomly, this amino group which binds to a dye molecule will loose its antigen binding activity. Even if the reactive dye reacts with the electron-riched atom(proton containing amino acid side chains) nearby antigen binding epitopes, the steric hindrance from dye molecule may still creat bias for antigen binding.

So, the direct staining reaction is designed to carry out in an acidic environment if the protein is an antibody, such that one dye molecule would react with the hydroxy groups from a amino acid side chain of the antibody, and the hydroxy groups on the oligosaccharide that conjugated to the antibody preferably than with a amino side chain. Or, a natural or synthetic water-soluble polymeric "carrier" is introduced to load the dye molecules and then conjugate the stained "carrier" to the olligosaccharide moiety of antibody preferably the oligosaccharide in the constant regions.

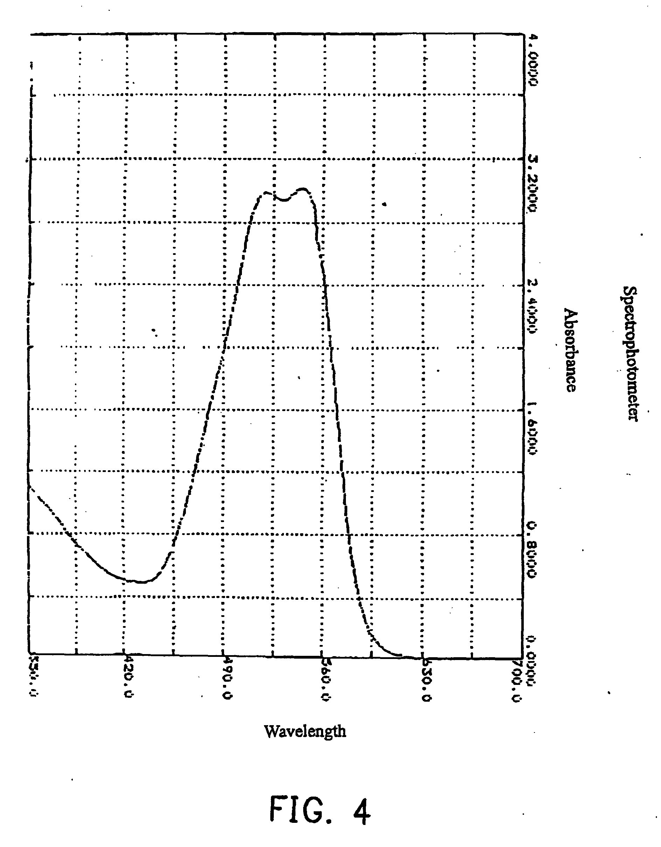

When the staining reaction is carried on to the normal non-antibody protein applicable to the immunoassay, there would not be much concern for loosing antigen-antibody binding affinity, as shown in Figure 4. However, care must still be taken to minize the possible blocking of the antibody-binding epitopes on protein antigen by the conjugating dye molecules.

The method of staining the antibody and its application to the immunoassay embodied in the present invention are concerned with the usage of reactive dye from the textile industry. An example of the reactive dye can be triazine series dyes, which are subject to nucleophilic substitution with a staining specimen, and vinyl sulfone series dyes, which are subject to nucleophilic addition with the staining specimen. Preferably, the reactive dye has a molecular weight (MW) of 600~800 Dalton (DA).

The triazine series reactive dyes has the following formula : 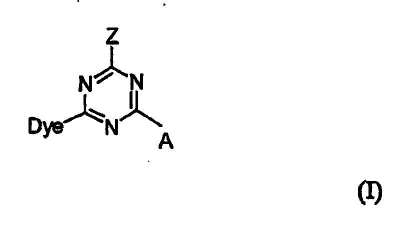 wherein A is alkyl or halogen such as chlorine or fluorine; Z is Cl or F; and Dye is a chromophor of the dye molecule.

When the triazine series reactive dye molecule binds to the protein by reacting with the hydroxy group, amino group, and thiol group of the amino acids on the antibody or antigen, a compound having the following formula is formed:

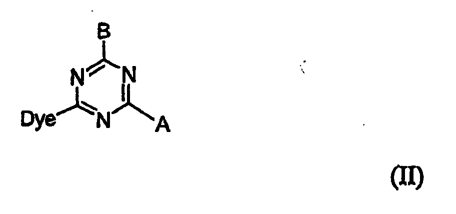

Wherein A is alkyl or halogen such as chlorine; Dye is a chromophor of the dye molecule; and B is NHR', OR' or SR', wherein R' is a moiety of the antibody or antigen.

The vinyl sulfone series dyes have the formula as follows:

Dye-C=C-S-O-O-Cl (III)

Wherin Dye is a chromophor of the dye molecule.

When the vinyl sulfone series reactive dye moleucle binds to the protein by reacting with the hydroxy group, amino group and thiol group of the amino acids in the antibody or antigen, a compound having the following formula is formed:

Dye-C=C-S-O-O-R (IV)

Wherein Dye is a chromophor of the dye molecule; R is NHR', OR' or SR', wherein R' is a moiety of the antibody or antigen.

With a control of pH value and salt selection, the reactive dye electrophilic reaction would occur primarily on hydroxy groups from the antibody and the hydroxy groups from the oligosaccharide that conjugates to the antibody, and left antigen-binding epitope untouched. With loading the stained carrier macromolecule on oligosaccharide of the Fc region, while maintaining the stained antibody of its original valence and immunological characteristics. After the stained protein is purified and separated to remove dye molecules that are not reacted, it can be applied to different purposes in the immunoassay. Thus, the method becomes a novel dye immunoassay, known as conjugate dye immunoassay (CDIA) and is more convenient than immunoassays. Also, the method is more stable, effective and sensitive than the enzyme immunoassay, while it is more convenient and less costly than FIA or LIA

The staining reactions for the glycosylated proteins, such as rabbit anti human myoglobin antibody and bovine serum albumin and the relevant tests results are described as followed:

### (a) The "direct" staining reaction for a myoglobin antibody

First, 10mg of rabbit antibody to human myoglobin was dissolved in a 0.01M sodium acetate buffer of pH 4.8, 0.2M sodium chloride solution. With 5mg of Procion red MX-8R being added, the solution mixture is stirred in a cold water bath in the dark. After a twenty-four hours reaction, the reacted solution mixture was injected into a Sephadex G-25 column with a dimension of 30 x 1.1cm. The column was then eluted at a rate of 20ml per hour using a 0.05M phosphate buffer of pH6.8. An eluent from the column was then collected using a graded tube, which collects 1ml in each tube. As the myoglobin antibody is stained by forming covalent bonding to the dye molecule, it has a large volume to prevent its entry into pores of molecular gel beads in the Sephadex G-25 column. So, the stained myoglobin antibody is eluted out first. The dye moleucles that do not react nor aggregate, on the other hand, enter the pores of the microporous gel beads and are washed out later. Thus, the tubes with stained myoglobin antibody show a characteristic red color and are collected for freeze drying before preserved for further research purposes.

### (b) The "carrier" staining and the binding of dye-loaded "carrier" to oligosaccharide moiety of myoglobin antibody

### (i) "carrier" staining

Bovine albumin was randomly selected as protein "carrier" for reactive dye staining so were the natural or synthetic water-soluble polymeric such as, dextran and polylysine. The active dye staining conditions of bovine albumin is described in section (f). And the staining conditions for these biopolymers varied from pH 10.5~12, and incubated from 1 to 3 hours at room temperature using Procion red MX-8R. The reactions were stoped by ammonium hydroxide and the mixtures were then subjected to the Sephadex G-25 columns with different dimensions from previous experiments. The columns were eluted at a rate of 30ml per hour using a 0.01M acetate buffer of pH5.5. The eluents were collected from the columns then either submitted to lyophilization for storagement or directly used in the following antibody conjugation experiments,

### (ii) Periodate activation of antibody oligosaccharide moiety and conjugation of stained "carrier" to activated myoglobin antibody

Antibodies re-equilibrated in reaction buffer (2.26 mg/ml in 0.1M sodium acetate, pH 5.5, 2mM *β* -mercaptoethanol) were oxidized by the addition of sodium periodate stock solution preapred in deionized water, to give a final concentration of periodate of 5mM and allowed to proceed for 30 minutes in cold room in the dark and terminated by adding 10 µl, 80mM sodium sulfite. The limited periodate treatment of glycoprotein such as antibody results in conversion of allyl hydroxyl group of the glycoprotein sugar moiety to an aldehyde group for further Schiff acid-base reaction with amino-group from dyed-loaded protein and polysine. To this was added 5ml solution of dye-loaded "carrier" in the presence of 0.01%(w/v) sodium cyanoborohydride. When dyed-loaded dextran (molecular weight approx. 10,000, clinical grade) was used as "carrier", the oxidized antibody which was further treated with 0.2ml, 0.05M ethylene diamine at pH 5.6. The ethylene diamine free reductive aminated antibody was then conjugated to dyed-loaded dextran which was also oxidized with periodate the same way as antibody oxidation, under the conditions same as before. A 1ml fraction of thus prepared dyed-loaded dextran-antibody mixture was applied onto a 35 x 1.1cm Sephadex G-150 column for separating unconjugated dyed-loaded dextran from the conjugate.

These stained "direct" and "carrier" conjugated myoglobin antibodies were lyophilized and reconstituted when needed in the estimated concentrations then submitted to further activity tests.

### (c) An assay for determining valence for the myoglobin antibody

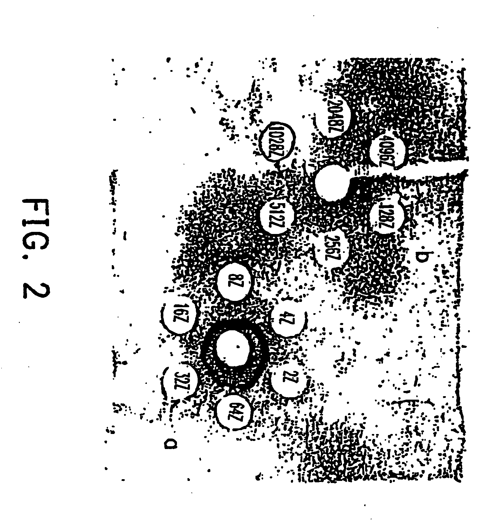

The method begins by preparing a 1% agarose gel in a 0.05M barbital buffer of pH8.2. The gel is dissolved at 50⁰C to prepare an Ouchterlony bi-directional immunodiffusion gel film having punched a line of holes, for the double diffusion immunoassay. Next, 15µl of human myoglobin having a concentration of 2.1mg/ml is placed in a central hole of the Ouchterlony agarose gel. And 15 *µ*l from unstained myoglobin antibody (1.43mg/ml) diluted in series of 2, 4, 8,16, 32, 64,128, 256, 512, 1024, 2048 and 4096 were respectively pippetted into peripheral holes surround the central hole in the Oucherlony agarose gel. The gel was incubated for six hours at room temperature before observing precipitation line appearing between myoglobin in the central hole and the diluted antibody preparation. The gel was submitted for further 6 hours incubation, however, no more precipitation lines were observed. And the gel was then stained using Amido black and de-stained in methanol/acetic acid solution. The condensed lines between the myoglobin and the unstained antibody diluted by a factor of 2-64 were Amido black stained antigen-antibody presipitation lines to show up as evidence in Fig. 2a and 2b.

The Ouchterlony bi-directional immunodiffusion were repeated with the stained antibodies. Both dye-loaded, stained "direct" and dye-loaded dextran "carrier" conjugated antibody having a concentration estimated to be 1.32mg/ml and 0.96mg/ml were then diluted by factors of 2, 4, 8, 16, 32 and 64 while the unstained antibody at 1.43mg/ml were also prepared with same set of dilutions as described. The antigen-antibody precipitation lines for stained "carrier", "direct" and unstained antibodies were shown as in Figure 3a, 3b, 3c and 3d, respectively. It appears that the stained "carrier" had higher sensitivity than stained "direct", under the current preparation conditions. In the absence of Amido black staining, there is only a blurry precipitation line appearing between the myoglobin in the central hole and the unstained myoglobin antibody diluted by a factor of 2~16, as shown in Figure 3c.

The observation made above show that the myoglobin antibody does not only maintain its activity, but also its valence as compared to the unstained antibody, after being reacted with the reactive dye in an environment of pH 4.8 as "direct" staining and dye-loaded "carrier" when dextran was used as "carrier" to conjugate myoglobin antibody.

### (d) A method of determining the binding region on the antibody for forming covalent bond with the reactive dye

### (i) Pretreatment of neuramidase sample

Neuramidase was dialzyed in 0.05M acetate buffer of pH5.6 at 4⁰C overnight, before it is applied onto a 5ml Sepharose 4B column immoblized with fetuin which is then washed with the buffer solution until its absorbance reduced to 0.05 at 210nm. The neuramidase is finally eluted out using bicarbonate buffer. After the neutralization of the neuramidase, it is made ready through a dialysis in acetate buffer again.

### (ii) Removal of oligosaccharide of myoglobin antibody

A 0.2ml portion of pre-treated neuramidase free of proteolytic enzyme, 5 unit; of jack bean α-mannosidase, 2 units of β-galactosidase and 10 units of calf liver α-glucosidase were mixed with 5ml of myoglobin antibody having a concentration of 1.46mg/ml, then Streptomycin is further added to make a 0.05% final concentration. The mixture above is incubated at 40⁰C for 48 hours, then 5ml of thus treated neuramidase sample is applied onto a Sephadex G-150 column having a dimension of 45 x 1.1 cm. The column is eluted at a rate of 10ml per hour, with a red flesh tint collected in each tube representing the myoglobin. After being ammonium sulfate precipitated and collected by a centrifuge, the redissolved myoglobin antibody provided for the following reactive dye staining reaction.

### (e) The staining reaction of the oligosaccharide of the myoglobin antibodies and the comparision of the native and deglycosylated antibodies staining results

The concentration of the native and deglycosylated, pre-treated antibody were adjusted to 1.0mg/ml, while the rest of the staining steps follow the same as those described in (f) for bovine albumin staining.

After G-25 fractionation, the red color breakthru fractions collected which contained Procion red MX-8R stained antobodies were pooled. The absorbance were measured of the diluted samples respectively using a Beckman DU-65 spectrophotometer. The result is shown in Table 1 below:

### Table 1; Aborbance of antibodies with and without removal of oligosaccharide moiety

As shown in Table 1, it clearly indicates that at least a part of Procion red MX-8R was bound by forming convalent bonds to the oligosaccharide portions of myoglobin antibody. With partial excision of the oligosaccharides from constant region and heavy chain portion of the antibody by the neuramidase and enymes mixture, the reactive dye molecules show a reduced reaction with the hydroxy group of the deglycosylated, pre-treated antibody, in an acidic environment as corresponded to results in Table 1.

### (f) The reactive dye staining preparation for bovine albumin

A bovine albumin of Cohn V grade having a concentration of 7.8mg/ml was selected for the staining reaction, for its propularity and availability. Since this staining reaction does not need to worry about occupancy of amino terminals by dye molecule to influence the binding activity of antibody to antigen, the reaction pH is adjusted to an alkaline range for maximal staining in the absence of denature albumin. Preferably, the pH is adjusted to about 10.5 to ensure reactions with the amino group, thiol group and hydroxy group on the amino acids that make the protein. Thus, this increases the staining efficiency as well as sensitivity when applied as labelled antigen in competitive immunoassay. Other staining and purification steps are similar to those described in (a). Fig. 4 shows the light abosrbance at visible region of the spectrum for the Sephadex G-25 purified dye-stained bovine albumin obtained from scanning result of an spectrophotometer,

### (g) The use of "direct" acid stained myoglobin antibody in sandwish-type immunochromatogrph

A 10 mg myoglobin antibody was stained and purified as described in (b). The Sephadex G-25 purified fraction was lyophilized and reconstituted at appropriate, estimated concentration in phosphate buffer, pH 8.0 for loading onto the conjugate pad of the immunnochromatrographic strip. Myoglobin of different concentration in reconstituted sera samples were used for testing and preparing a calibration curve.

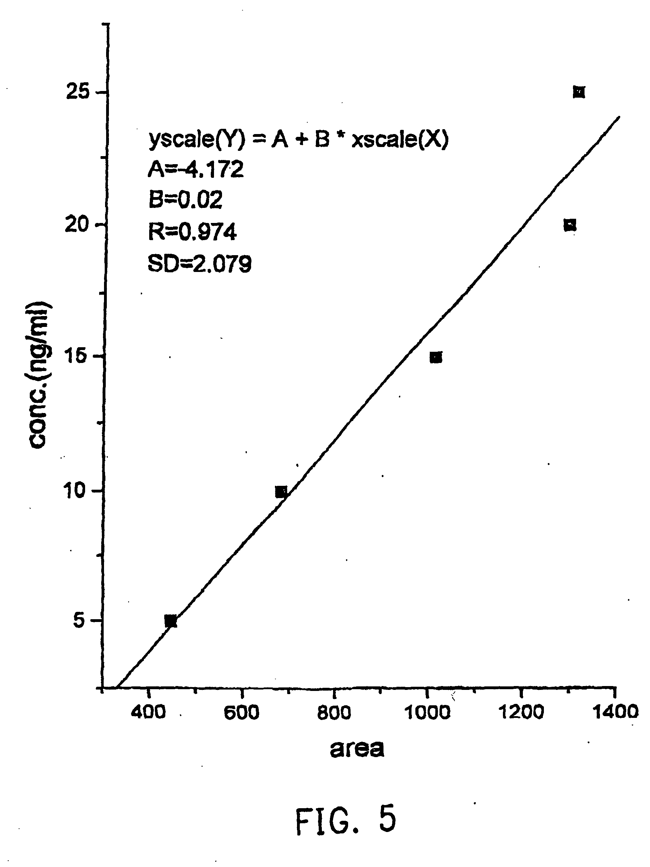

While Fig. 5 shows the result of immunochromatograph for myoglobin when used "direct" stained antibody in sandwish-type immunoassay.

It is understood from the experiments and tests above that when the staining reaction is carried out in the non-antibody protein, the reaction can occur in the alkaline environment without a need for high temperature, salt or electrolyte. Under such easily manipulated situation, the amino group, thiol group and hydroxy group on the amino acid side chain can all show an electro-enriched state, which enhances a nucleophilic reaction of the dye molecule. As a result, a stained protein conjugate with covalent bonds is formed to obtain a higher staining intensity for the protein and retain its natural conformation. But, when the staining reaction is carried out on an antibody, the reaction is selected to occur in the acid environment, so that dye moleucles react with the hydroxy groups from amino acid side chain of antibody and hydroxy groups from the constant region and heavy chain. This protects the antigen binding ability of the antibody through its amino groups, as the amino groups of the antibody are not bound with the dye molecules. Therefore, the sensitivity of the immunoassay is significantly improved.

According to the present invention, the stained protein can serve as a labeled ligand that applies to enzyme linked immunoassay, histology, cell cytology, blotting, all kinds of immunoaggulutination, all kinds of electrophoresis, all kinds of immunochromatography, all kinds of liquid chromatography, biosensors, biochips and methods of locating and quantifying targets using color in different kinds of nucleic acid probes or other chemical analyses.

The present embodiment has been described using the column separation method as an example to separate the unreacted dye molecules from stained protein molecules. However, other separating methods, such as molecular sieve, filtering membrane, salting out, or organic solvent can also be understood by one skill in the art to separate the dissociated dye molecules form stained protein molecules after the protein is stained with the reactive dye. Once the proteins stained with the reactive. dye, those dye molecules that do not react with the protein can be removed using the separation method above. Next, different stained protein can be purified to show a high stability, while it is freeze-dried in a vaccum to improve its storage.

The importance of the present invention is illustrated in Fig. 1. When the ligand L is a radioactive isotope, enzyme, fluorescent or luminescent element, the applications appear to be RIA, EIA, FIA or LIA. With the same mechanism, when the ligand L is substituted with reactive dye molecule in the invention, a conjugate dye immunoassay, (CDIA) may be developed to replace the EIA, other conventional immunoassay, chemical and biochemical analyses, since the conjugate dye analysis, CDA has the following potential advantages and applicaitons:

(i) It is more sensitive than the enzyme immunoassay since the dye molecules are designed and selected with high molecular absorbance.

(ii) It has a shorter duration of immunoassay than EIA, since it alleviate the time waste in enzyme-substrate incubation.

(iii) The protein is more stable after binding to dye molecules with covalent bonds, but it has to be careful for not loosing its antigen-antibody activity by overloading of the protein molecules.

(iv) The procedure is straight forward and easy with mass production, only a control of pH with simple separation and purification is required to obtain a useful stained protein for antigen antibody interaction.

(v) Since the reactive dye is consumed in huge amount in the textile industry, it is usually sold by tons. Therefore, it implies a comparable lower cost than any other ligands for immunoassay.

(vi) With a wide variety of dye color choices available, the applicability of the stained protein can be greatly expanded in the field of biochemical analysis.

(vii) The sensitivities of the CDIA shall be greatly enhanced if the materials and processes of both "direct" acid staining and dye-loaded "carrier" staining are optimized.

(viii) The idea of dye-loaded "carrier" can naturally be extended to small molecular antigen competitive immunoassay and other immunoassays of many different applications, when both the dye and small organic molecule antigen are'loaded' on the natural or synthetic water-soluble polymeric carrier.

(viv) The very same idea of dye-loaded "carrier" can naturally be extended to molecular probe applications, when both the dye and specific molecular probe are 'loaded' on the natural or synthetic water-soluble polymeric carrier.

It will be apparent to those skilled in the art that various modifications and variations can be made to the structure of the present invention without departing from the scope or spirit of the invention. In view of the foregoing, it is intended that the present invention cover modifications and variations of this invention they fall within the scope of the following claims and their equivalent.

## Claims

1. A method for staining a water soluble protein, the method is **characterized by** reacting a reactive dye with a water soluble protein for carrying out a staining reaction, wherein the reactive dye is selected from a group consisting of a triazine series reactive dye and a vinyl sulfone series reactive dye or a mixed of triazine and vinyl sulfone functional groups series reactive dye used in a textile industry, and the staining reaction is carried out in an acidic or alkaline environment;
wherein the staining reaction is carried out in the acidic environment if the water soluble is an antibody a glycoprotein, so that a dye molecule would react with a hydroxy group from an amino acid side chain of the protein, a hydroxy group from the constant region of the antibody, a hydroxy group from a heavy chain of the antibody, and a hydroxy group from an oligosaccharide conjugated to the antibody.

2. The method of claim 1, wherein the alkaline environment has a pH value of about 10~12.

3. The method of claim 1, wherein the staining reaciton is carried out in an acidic environment having a pH value of 4~6 when the antibody is a myoglobin antibody.

4. The method of claim 1, further comprising separating unreacted and hydrolyzed dye molecules form a stained protein using a column separation method, a molecular sieve, a filtering membrane, a dialysis or an organic solvent, after the step of reacting a reactive dye with the water soluble protein.

5. The method of claim 1, wherein the water-soluble molecule stained as recited above can be applicable to an enzyme linked immunoassay.

6. The method of claim 1, wherein the water-soluble molecule stained as recited above can be applicable to locating and quantifying targets using color in histology.

7. The method of claim 1, wherein the water-soluble molecule stained as recited above can be applicable to locating and quantifying targets using color in cell cytology.

8. The method of claim 1, wherein the water-soluble molecule stained as recited above can be applicable to locating and quantifying targets using color in all kinds of blotting.

9. The method of claim 1, wherein the water-soluble molecule stained as recited above can be applicable to locating and quantifying targets using color in all kinds of immunoagglutination.

10. The method of claim 1, wherein the water-soluble molecule stained as recited above can be applicable to locating and quantifying targets using color in all kinds of electrophoresis.

11. The method of claim 1, wherein the water-soluble molecule stained as recited above can be applicable to locating and quantifying targets using color in all kinds of immunoassay.

12. The method of claim 1, wherein the water-soluble molecule stained as recited above can be applicable to all kinds of liquid chromatography.

13. The method of claim 1, wherein the water-soluble molecule stained as recited above can be applicable to all kinds of biosensors.

14. The method of claim 1, wherein the water-soluble molecule stained as recited above can be applicable to all kinds of protein chips.

15. The method of claim 1, wherein the water-soluble molecule stained as recited above can be applicable to locating and quantifying using all kinds of nucleic acid probes.

16. The method of claim 1, wherein the water-soluble molecule stained as recited above can be applicable to analyses.

17. A method for staining selectively a glycoprotein particularity an antibody, the method is **characterized by** reacting a reactive dye with natural or synthetic water-soluble polymer for carrying out the conjugation of the dye-loaded "carrier" to the oligosaccharide moiety of the antibody molecule selectively after periodate activation treatment, the staining reaction being carried out in an alkaline environment, the conjugation reaction being carried out in an appropriate covalent bond forming environment,
wherein the staining reaction is carried out in the alkaline environment if the water soluble is a natural or synthetic water-soluble polymer, so that a dye molecule would react with a hydroxy group or an amino group from a natural or synthetic water-soluble polymer then the stained polymer can be conjugated to the activated oligosaccharide moiety of antibody molecules in an appropriate covalent bond-forming reaction;
wherein the reactive dye is selected from a group consisting of a triazine series reactive dye and a vinyl sulfone series reactive dye or a mixed of triazine and vinyl sulfone functional groups series reactive dye used in a textile industry;
wherein, the staining reaction is carried out to the natural or synthetic water-soluble polymeric carrier before it is conjugated to antibody molecule, so that a dye molecule would react with a electron riched functional group in the natural or synthetic water-soluble polymer, in any feasible number to enhance staining intensity, i.e., immunoassay sensitivities, under manipulatable conditions without interfering the antigen-antibody reaction.

18. The method of claim 17, wherein the reactive dye is directly replaced by a enzyme.

19. The method of claim 17, wherein the chromogen on the reactive dye is replaced by a fluorophor.

20. The method of claim 17, wherein the reactive dye is directly replaced by a fluorescent or lumninescent element, when staining of oligosacchride approach is uptaken.

21. The method of claim 17, wherein the reactive dye can be directly replaced by a radioactive nuclide.

22. The method of claim 17, wherein the reactive dye is directly replaced by an electroactive element, which can generate either ampermotric or conductometric or voltametric signals.

23. The method of claim 17, wherein the part of the reactive dye can be replaced by a small molecular antigen.

24. The method of claim 17, wherein the part of the reactive dye can be replaced by a molecular probe such as a nucleic acid probe.

25. The method of claim 17, further comprising separating unreacted and hydrolyzed dye molecules form a conjugated protein using a column separation method, a molecular sieve, a filtering membrane, a dialysis or an organic solvent, after the step of conjugating a dye-loaded "carrier" with activated glycoprotein.

26. The method of claim 17, wherein the dye-loaded "carrier" conjugated with activated glycoprotein as recited above can be applicable to an enzyme linked immunoassay.

27. The method of claim 17, wherein the dye-loaded "carrier" conjugated with activated glycoprotein as recited above can be applicable to locating and quantifying targets using color in histology.

28. The method of claim 17, wherein the dye-loaded "carrier" conjugated with activated glycoprotein as recited above can be applicable to locating and quantifying targets using color in cell cytology.

29. The method of claim 17, wherein the dye-loaded "cariier" conjugated with activated glycoprotein as recited above can be applicable to locating and quantifying targets using color in all kinds of blotting.

30. The method of claim 17, wherein the dye-loaded "carrier" conjugated with activated glycoprotein as recited above can be applicable to locating and quantifying targets using color in all kinds of immunoagglutination.

31. The method of claim 17, wherein the dye-loaded "carrier" conjugated with activated glycoprotein as recited above can be applicable to locating and quantifying targets using color in all kinds of electrophoresis.

32. The method of claim 17, wherein the dye-loaded "carrier" conjugated with activated glycoprotein as recited above can be applicable to locating and quantifying targets using color in all kinds of immunoassay.

33. The method of claim 17, wherein the dye-loaded "carrier" conjugated with activated glycoprotein as recited above can be applicable to all kinds of liquid chromatography.

34. The method of claim 17, wherein the dye-loaded "carrier" conjugated with activated glycoprotein as recited above can be applicable to all kinds of biosensors.

35. The method of claim 17, wherein the dye-loaded "carrier" conjugated with activated glycoprotein as recited above can be applicable to all kinds of protein chips.

36. The method of claim 17, wherein the dye-loaded "carrier" conjugated with activated glycoprotein as recited above can be applicable to locating and quantifying using all kinds of nuclei acid probes.

37. The method of claim 17, wherein the dye-loaded "carrier" conjugated with activated glycoprotein as recited above can be applicable to analyses.

38. The method of claim 17, wherein the natural or synthetic water-soluble polymer can be protein, dextran, polylysine, poly glutamic acid, poly lactic acid, polyacrylamide, etc.

39. The method of claim 17, wherein the staining reaction is carried out in an alkalilne environment having a pH value of 10~12 when the water-soluble is dextran or polylysine.

40. The method of claim 17, further comprising separating unreacted and hydrolyzed dye molecules form a stained natural or synthetic water-soluble polymer using a precipitation method, a molecular sieve, a filtering membrane, a dialysis or an organic solvent, after the step of reacting a reactive dye with natural or synthetic water-soluble polymer.

41. The method of claim 17, wherein the conjugation between oxidized "carrier" and aminated glycoprotein can also be replaced in an over-oxidized form when performing periodate oxidation to generate carboxylic functional group for N-hydroxysuccimide treatment and further condensation with amine functional group from other molecule in the presence of carbodiimide.

42. The method of claim 17, wherein the conjugate reaction is carried out in Schiff acid-base reaction.

43. A method for staining water-soluble protein, the method is **characterized by** reacting a reactive dye with the water soluble protein for carrying out a staining reaction, with the reactive dye is selected from a group consisting of a triazine series reactive dye and a vinyl sulfone series dye or a mixed of triazne and vinyl sulfone functional groups series reactive dye used in a textile industry, and the staining reaction is carried out in an acidic or alkaline environment;
wherein the staining reaction is carried out in the acidic environment if the water soluble protein requires protection of an amino group, so that a dye molecule would react with a hydroxy group preferentially from a amino acid side chain of the water-soluble protein and a hydroxy group from an oligosaccharide conjugated to the water-soluble protein.

44. The method of claim 43, wherein the alkaline environment has a pH value of 10~12.

45. The method of claim 43, wherein the staining reaction is carried out in an acidic environment having a pH value of 4~6 when the water-soluble protein that requires protection of the amino group is a myoglobin antibody.

46. The method of claim 43, further comprising separating unreacted and hydrolyzed dye molecules form a stained protein using a column separation method, a molecular sieve, a filtering membrane, a dialysis or an organic solvent, after the step of reacting a reactive dye with the water soluble protein.

47. The method of claim 43, wherein the water-soluble molecule stained as recited above can be applicable to an enzyme licked immunoassay.

48. The method of claim 43, wherein the water-soluble molecule stained as recited above can be applicable to locating and quantifying targets using color in histology.

49. The method of claim 43, wherein the water-soluble molecule stained as recited above can be applicable to locating and quantifying targets using color

50. The method of claim 43, wherein the water-soluble molecule stained as recited above can be applicable to locating and quantifying targets using color in cell cytology.

51. The method of claim 43, wherein the water-soluble molecule stained as recited above can be applicable to locating and quantifying targets using color in all kinds of blotting.

52. The method of claim 43, wherein the water-soluble molecule stained as recited above can be applicable to locating and quantifying targets using color in all kinds of immunoagglutination.

53. The method of claim 43, wherein the water-soluble molecule stained as recited above can be applicable to locating and quantifying targets using color in all kinds of electrophoresis.

54. The method of claim 43, wherein the water-soluble molecule stained as recited above can be applicable to locating and quantifying targets using color in all kinds of immunoassay.

55. The method of claim 43, wherein the water-soluble molecule stained as recited above can be applicable to all kinds of liquid chromatography.

56. The method of claim 43, wherein the water-soluble molecule stained as recited above can be applicable to all kinds of biosensors.

57. The method of claim 43, wherein the water-soluble molecule stained as recited above can be applicable to all kinds of protein chips.

58. The method of claim 43, wherein the water-soluble molecule stained as recited above can be applicable to locating and quantifying using all kinds of nuclei acid probes.

59. The method of claim 43, wherein the water-soluble molecule stained as recited above can be applicable to analyses.
